# EUROPÄISCHE PATENTSCHRIFT

(11) **EP 0 627 412 B1**
(45) Veröffentlichungstag und Bekanntmachung des Hinweises auf die Patenterteilung: **20.08.1997**
(21) Anmeldenummer: 94105113.8
(22) Anmeldetag: 31.03.1994
(51) Int. Cl.: C07C 255/19, C07C 253/30

(54) **Verfahren zur Herstellung von 2-Cyanoacetoxy-propionsäureestern**
Process for the preparation of 2-cyanoacetoxypropionic acid esters
Procédé de préparation d'esters de l'acide de 2-cyanoacétoxy propionique

(30) Priorität: 03.06.1993 DE 4318381
(43) Veröffentlichungstag der Anmeldung: 07.12.1994
(73) Patentinhaber: HÜLS AKTIENGESELLSCHAFT, 45764 Marl (DE)
(72) Erfinder: Kaufhold, Manfred, Dr., D-45770 Marl (DE); Feld, Marcel, Dr., D-51147 Köln (DE)

(56) Entgegenhaltungen:
- EP-A- 0 127 855
- CHEMICAL ABSTRACTS, vol. 102, no. 24, 1985, Columbus, Ohio, US; abstract no. 204452h; & JP-A-59 222 461

## Beschreibung

Die Erfindung betrifft ein Verfahren zur Herstellung von 2-Cyanoacetoxypropionsäureestern, insbesondere auch solcher mit hoher optischer Reinheit der allgemeinen Formel III, durch Umsetzung von Cyanessigsäureestern I mit Milchsäureestern, insbesondere optisch aktiven Milchsäureestern II, in Gegenwart eines Katalysators unter Abspaltung des Alkoholrestes aus dem Cyanessigsäureester.

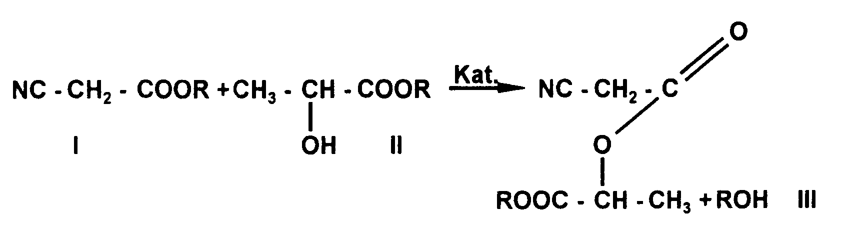

Es sind verschiedene Verfahren zur Herstellung von III literaturbekannt. So schlägt die EP-A 0 127 855 als Synthese für III eine Kondensation von Cyanessigsäure mit Milchsäureestern unter Dehydratisierung vor. Welche chemische Substanzen hierfür benötigt werden, wird nicht erwähnt. Auf jeden Fall sind nur kostspielige Chemikalien einsetzbar, da die Dehydratisierung unter schonenden Bedingungen stattfinden muß, weil sonst hochsiedende Verbindungen als Hauptprodukt gebildet werden.

Dieses Verfahren ist also relativ aufwendig und unwirtschaftlich. Ein weiteres Problem ist die thermische Unbeständigkeit der Cyanessigsäure. Bei etwas höheren Temperaturen, schon unter 100 °C, fängt sie an sich zu zersetzen, und bei noch höheren Temperaturen kann diese Zersetzung explosionsartig erfolgen, d. h. bei diesem Verfahren spielen auch Sicherheitsprobleme eine wichtige Rolle.

Ein weiteres Verfahren gemäß japanischem Patent JP 84,222,461 geht ebenfalls von Cyanessigsäure aus und setzt diese mit entsprechenden α-chlorierten Estern um, das wäre im Falle des erfindungsgemäßen Verfahrens ein α-Chlorpropionsäureester IV.

Diese sind aber unter Reaktionsbedingungen ebenfalls instabil, so daß bei diesem Syntheseweg 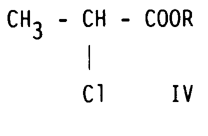 beide Einsatzprodukte instabil wären.

Eine technische Realisierung ist unter solchen Voraussetzungen sehr problematisch. Diese wenigen Verfahren benötigen teure Chemikalien, liefern unbefriedigende Ausbeuten und besitzen außerdem noch Sicherheitsprobleme. Wünschenswert ist daher ein einfaches Verfahren, bei dem man von stabilen, leicht zugänglichen Einsatzstoffen ausgeht, wie z. B. von den Estern, und diese in Gegenwart eines Katalysators ohne zusätzliche Chemikalien zum Zielprodukt umsetzt.

Es besteht ein großes Interesse an einem derartigen Verfahren, nach dem man bei geringem technischen Aufwand und ohne den Einsatz von teuren Chemikalien die Ester der Cyanessigsäure und der Milchsäure in Gegenwart eines Katalysators unter Abspaltung eines Alkohols umsetzt und das Zielprodukt, den 2-Cyanacetoxy-propionsäureester, durch einfache Destillationen gewinnt, weil diese Produkte wichtige Rohstoffe für den Klebestoffsektor (s. genanntes EP-A 0 127 855) und für den Pharmabereich sind.

Als Einsatzstoffe für Pharmaprodukte ist hohe optische Reinheit wünschenswert, d. h. die optische Information, die man beim Einsatz von optisch aktiven Milchsäureestern in das System einbringt, soll weitgehend erhalten bleiben.

Die sich hieraus ergebende Aufgabe wird erfindungsgemäß entsprechend den Angaben der Patentansprüche gelöst.

Gegenstand der Erfindung ist ein Verfahren zur Herstellung von 2-Cyanoacetoxy-propionsäureestern der allgemeinen Formel III 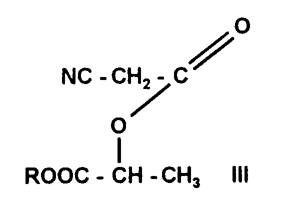 welches dadurch gekennzeichnet ist, daß man Cyanessigsäureester der allgemeinen Formel I unter Abspaltung von Alkohol mit Milchsäureestern der allgemeinen Formel II 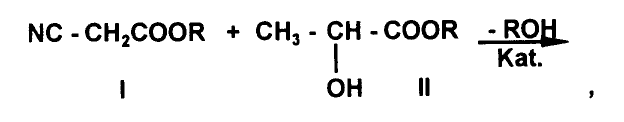 wobei R einen C₁-C₁₀-Kohlenwasserstoffrest bedeutet und 1 bis 20 Mol Cyanessigsäureester pro Mol Milchsäureester eingesetzt werden. Die Reaktion erfolgt in Gegenwart von 0,001 bis 0,1 Mol eines basischen Katalysators pro Mol Milchsäureester bei 100 bis 230 °C unter Abdestillieren des entsprechenden Alkohols, wobei sie vor Erreichen der theoretisch möglichen Alkoholmenge bei einem Milchsäureumsatz von 76 bis 85 % dadurch abgebrochen wird, daß man den Katalysator zerstört und unwirksam macht.

Überraschenderweise erhält man reine 2-Cyanoacetoxy-propionsäureester, wenn man einen Cyanessigsäureester in Gegenwart eines Metallalkoholats als Katalysator mit einem Milchsäureester umsetzt, wobei der entsprechende Alkohol aus dem Cyanessigsäureester abgespalten wird. Ein wichtiges Merkmal der vorliegenden Erfindung ist, daß diese Reaktion nicht bis zum Ende durchgeführt wird, d. h. bis die theoretisch mögliche Menge an Alkohol angefallen ist, sondern die Reaktion muß nach einem bestimmten Zeitpunkt abgebrochen werden, indem man den Katalysator, z. B. durch Ansäuern, zerstört und unwirksam macht.

Wird die Reaktion nicht abgebrochen, entstehen hauptsächlich Hochsieder, vermutlich Polyester, die eine Gewinnung der Zielprodukte unmöglich oder zumindest unwirtschaftlich machen. Den Verlauf der Reaktion kann man leicht mit gaschromatographischen Analysen verfolgen. Man erkennt dann, daß ab einem Gehalt von z. B. 50 bis 60 % an 2-Cyanoacetoxy-propionsäureester vermehrt Hochsieder gebildet werden. Das ist dann der richtige Zeitpunkt zum Abbruch der Reaktion. Es ist sehr überraschend, daß die Reaktion der beiden Ester überhaupt eine so hohe Selektivität zum Zielprodukt hat. Wegen der mannigfaltigen Reaktionsmöglichkeiten sollte man vielmehr vermuten, daß direkt wertlose Polyester entstehen, z. B. indem Milchsäureestermoleküle selbst miteinander reagieren.

Der gefundene Effekt war nicht vorhersehbar. Die Vorteile des Verfahrens sind seine einfache Durchführbarkeit und seine gute Wirtschaftlichkeit.

Die praktische Durchführung der Umsetzung und der Aufarbeitung erfolgt beispielsweise in der folgenden Weise:

Man verwendet eine Rührapparatur mit Destillationskolonne und Destillationsbrücke. Die beiden Ester werden vorgelegt und unter Rühren der Katalysator zugegeben. Vorteilhaft - besonders bei größeren technischen Mengen sowie bei kontinuierlicher Fahrweise - ist es, den Cyanessigsäureester vorzulegen, Katalysator zuzugeben und dann den Milchsäureester zu dosieren. Das Molverhältnis Milchsäureester zu Cyanessigsäureester beträgt vorzugsweise 1 : 1 bis 1 : 10, insbesondere 1 : 1,1 bis 1 : 2, d. h. Cyanessigsäureester wird bevorzugt im Überschuß eingesetzt. Das Molverhältnis Katalysator zu Milchsäureester beträgt vorzugsweise 0,005 : 1 bis 0,05 : 1, insbesondere 0,01 : 1 bis 0,05 : 1.

Als Katalysatoren verwendet man basische Verbindungen, wie z. B. Metallalkoholate, vorzugsweise der Alkali- und Erdalkalimetalle, wie Na, K, Mg, Ca, Sr, Ba und als Alkohole C₁ - bis C₁₀-Alkanole geradkettig und/oder verzweigt, aus wirtschaftlichen Gründen besonders Natrium- oder Kaliummethylat, -ethylat oder -butylat. Man kann aber auch Verbindungen einsetzen, die Alkoholate bilden oder zunächst mit der Hydroxylgruppe des Milchsäureesters reagieren, wie Natriumhydrid, Natriumamid, Grignardverbindungen usw. Nach dem Vermischen der Komponenten wird das Gemisch erwärmt und der sich dann bildende Alkohol abdestilliert. Anschließend wird die Reaktionstemperatur allmählich erhöht, so daß ständig Destillat anfällt.

Die Reaktion läuft vorzugsweise bei 120 bis 200 °C, insbesondere bei 140 bis 200 °C, ab. Während der Destillation werden ständig Proben aus dem Reaktor gezogen und gaschromatographisch schnell analysiert, z. B. nach 6, 8, 10 und 12 Stunden. Wenn die Analysen zeigen, daß der 2-Cyanoacetoxypropionsäureester im Gehalt nicht mehr, dagegen die Hochsieder aber zunehmen, wird abgestellt. Die Reaktionszeit kann auch in einem Vorversuch zunächst ermittelt werden, um bei der Produktion mehr Sicherheit zu haben und besser planen zu können.

Nach dem Abkühlen wird angesäuert, z. B. mit Phosphorsäure, Schwefelsäure, Salzsäure, Salpetersäure oder einer organischen Carbonsäure, wie Ameisensäure, Essigsäure usw. und dann der Ansatz destillativ aufgearbeitet. Hierbei ist es vorteilhaft, aber nicht unbedingt erforderlich, zunächst die Reaktionsprodukte und die nicht umgesetzten Einsatzprodukte vom hochsiedenen Rückstand abzutoppen und dafür ein hochsiedendes Lösungsmittel zuzusetzen, das den Rückstand dünnflüssiger macht. Als Lösungsmittel sind eine Vielzahl von chemischen Substanzen geeignet, die hoch genug sieden und unter den Bedingungen stabil sind, wie z. B. Weißöle, Paraffine, Alkylaromaten usw., aus wirtschaftlichen Gründen z. B. Wärmeträger wie MARLOTHERM S usw. Das vom Rückstand befreite Destillat wird anschließend durch fraktionierte Destillation gereinigt.

Die 2-Cyanoacetoxy-propionsäureester setzt man beispielsweise zur Herstellung von Klebstoffen ein (s. o. EP). Außerdem sind diese Ester wichtige Zwischenprodukte zur Herstellung von Pharma-Produkten.

Den folgenden Beispielen ist weiteres Zahlenmaterial zu entnehmen. Sie sollen das erfindungsgemäße Verfahren näher erläutern.

### Beispiel 1

Man benutzt eine Glasapparatur, die aus einem Dreihalskolben mit Rührer, Thermometer, Destillationskolonne und Destillationsbrücke mit Vorlage besteht.

Man setzt ein:
- 509 g (5,14 Mol): Cyanessigsäuremethylester
- 437 g (4,2 Mol): L(-)Milchsäuremethylester
- 14 g: Natriummethylat, gelöst in Methanol (30%ig)

Die Produkte werden vorgelegt, vermischt und erwärmt. Ab ca. 140 °C, besonders ab 150 °C, fällt Methanol an, das abdestilliert wird. Damit ständig Destillat anfällt, wird die Reaktionstemperatur allmählich erhöht, bis ca. 200 °C. Nach 6, 8, 10 und 12 Stunden werden Proben aus dem Reaktor gezogen und analysiert. Die GC-Analysen zeigen folgenden Reaktionsverlauf:

| Probe Nr. | MME | CEME | CPSM | UBK |
|---|---|---|---|---|
| 1 (nach 6 h) | 30 | 44 | 25 | 0,2 |
| 2 (nach 8 h) | 24 | 39 | 36 | 0,4 |
| 3 (nach 10 h) | 16 | 31 | 51 | 0,7 |
| 4 (nach 12 h) | 11 | 27 | 57 | 0,9 |
| Abkürzungen: MME = Milchsäuremethylester CEME = Cyanessigsäuremethylester UBK = unbekannte, hochsiedende Komponente | | | | |

Nach der Reaktionszeit von 12 Stunden wird die Reaktion abgebrochen, indem abgekühlt und mit 6,0 g Phosphorsäure (85 %ig) angesäuert wird. Der Methanolanfall beträgt 140 g.

Um leichter vom hochsiedenden Rückstand abtoppen zu können, wird zum Reaktionsprodukt 300 g MARLOTHERM S zugegeben und an einer Destillationsbrücke im Vakuum bei 0,2 mbar und einem Temperaturbereich von 42 bis 164 C abgetoppt.

Es werden 666 g Destillat erhalten, das CPSM in einer Konzentration von ca. 57 % enthält. Durch fraktionierte Destillation erhält man bei 13 mbar in einem Temperaturbereich von 153 bis 155 °C CPSM in 96%iger Reinheit.
Der Umsatz an Milchsäureester liegt bei 79 % und die Ausbeute an destilliertem CPSM, bezogen auf umgesetzten Ester, bei 62 % d. Th. Die optische Drehung beträgt -54°.

### Vergleichsbeispiel

Man verwendet die im Beispiel 1 benutzte Apparatur und setzt die hier genannten Produkte ein.
Die Durchführung erfolgt wie im Beispiel 1 beschrieben, mit dem Unterschied, daß man nicht die laufende Reaktion unterbricht, z. B. nach 12 Stunden, sondern wie sonst üblich durchlaufen läßt, und zwar 17 Stunden. Es ergibt sich folgender Reaktionsverlauf:

### GC-Analysen der Laufproben

| Probe Nr. | MME | CEME | CPSM | UBK |
|---|---|---|---|---|
| 1 (nach 6 h) | 30 | 42 | 25 | 0,2 |
| 2 (nach 7,5 h) | 23 | 37 | 37 | 0,4 |
| 3 (nach 10 h) | 17 | 31 | 49 | 0,7 |
| 4 (nach 12 h) | 11 | 25 | 59 | 0,9 |
| 5 (nach 14 h) | 10 | 20 | 59 | 1,4 |
| 6 (nach 16 h) | 12 | 18 | 53 | 3,0 |
| 7 (nach 17 h) | 13 | 17 | 50 | 4,0 |

Das Reaktionsprodukt besteht aus einer hochviskosen, klebrigen, dunkelbraunen Masse, die man mit üblichen einfachen Techniken nicht aufarbeiten kann, d. h. diese Arbeitsweise führt nicht zum Ziel.

Im Vergleich zum Beispiel 1 sieht man, daß die Reaktion viel früher abgebrochen werden muß.

### Beispiele 2 und 3

Man verwendet die im Beispiel 1 benutzte Apparatur, setzt die hier genannten Produkte ein, mit dem Unterschied, daß die Einsatzmenge an Cyanessigsäuremethyleter in einem Fall verkleinert wird auf ein Molverhältnis der Ester wie 1 : 1 und im anderen Fall vergrößert wird auf ein Molverhältnis wie 1 : 10. Im ersten Fall erhält man eine Ausbeute an CPSM von 45 % und im zweiten Fall von 72 % d. Th., bezogen auf umgesetzten Milchsäureester. Die Umsätze an diesem Ester liegen bei 76 bzw. 81 %.

### Beispiele 4 und 5

Man verwendet die im Beispiel 1 benutzte Apparatur, setzt die hier genannten Produkte an, aber mit dem Unterschied, daß die Einsatzmenge an Katalysator in einem Fall verkleinert wird auf ein Molverhältnis Natriummethylat zu Milchsäureester wie 0,01 : 1 und im anderen Fall vergrößert wird auf ein Molverhältnis 0,05 : 1.

Die günstigen Zeitpunkte zum Abbruch der Reaktion werden wieder mittels gaschromatographischer Analytik ermittelt.

Im ersten Fall wird die Reaktion nach 15 Stunden abgebrochen. Der Umsatz an Milchsäureester liegt bei 85 % und die Ausbeute an destilliertem CPSM, bezogen auf umgesetzten Ester, bei 48 % d. Th. Im zweiten Fall wird die Reaktion nach 10 Stunden abgebrochen. Der Umsatz an Milchsäureester liegt bei 83 % und die Ausbeute an destilliertem CPSM, bezogen auf umgesetzten Ester, bei 53 % d. Th. Die Beispiele zeigen also, daß der günstigste Zeitpunkt zum Reaktionsabbruch bei Veränderung der Reaktionsbedingungen jeweils ermittelt werden muß.

### Beispiel 6

Apparatur, Durchführung und Einsatzprodukte wie bei Beispiel 1, mit dem Unterschied, daß man an Stelle von Natriummethylat Kaliummethylat einsetzt. Die Reaktion wird nach 11 Stunden abgebrochen.

Die übrigen Ergebnisse sind vergleichbar wie beim Beispiel 1.

## Patentansprüche

1. Verfahren zur Herstellung von 2-Cyanoacetoxy-propionsäureestern der allgemeinen Formel III 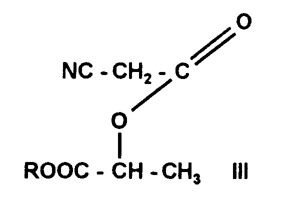 dadurch gekennzeichnet,
daß man Cyanessigsäureester der allgemeinen Formel I unter Abspaltung von Alkohol mit Milchsäureestern der allgemeinen Formel II 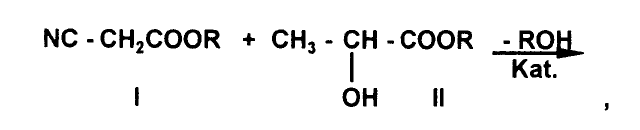 wobei R einen C₁-C₁₀-Kohlenwasserstoffrest bedeutet und 1 bis 20 Mol Cyanessigsäureester pro Mol Milchsäureester eingesetzt werden, in Gegenwart von 0,001 bis 0,1 Mol eines basischen Katalysators pro Mol Milchsäureester bei 100 bis 230 °C unter Abdestillieren des entsprechenden Alkohols, umsetzt und die Reaktion vor Erreichen der theoretischen Alkoholmenge bei einem Milchsäureesterumsatz von 76 bis 85 % dadurch abbricht, daß man den Katalysator zerstört und unwirksam macht.

2. Verfahren nach Anspruch 1,
dadurch gekennzeichnet,
daß man Reaktionstemperaturen von 140 bis 200 °C wählt.

3. Verfahren nach den Ansprüchen 1 und 2,
dadurch gekennzeichnet,
daß man pro Mol Milchsäureester 1 bis 10 und bevorzugt 1,1 bis 2 Mol Cyanessigsäureester einsetzt.

4. Verfahren nach den Ansprüchen 1 bis 3,
dadurch gekennzeichnet,
daß man als Katalysatoren Alkoholate von Alkalimetallen oder Erdalkalimetallen, vorzugsweise von Na, K, Mg, Sr, Ba und als Alkohole C₁- bis C₁₀-geradkettige oder verzweigte Alkanole einsetzt, vorzugsweise Na- oder K-methylat, -ethylat oder -butylate.

5. Verfahren nach den Ansprüchen 1 bis 4,
dadurch gekennzeichnet,
daß man je Mol Milchsäureester 0,005 bis 0,05, insbesondere 0,01 bis 0,05 Mol, Katalysator einsetzt.

## Claims

1. A process for the preparation of 2-cyanoacetoxy-propionic esters of the general formula III 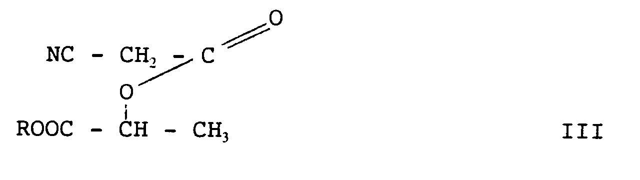 characterized in that cyanoacetic esters of the general formula I are reacted with lactic esters of the general formula II 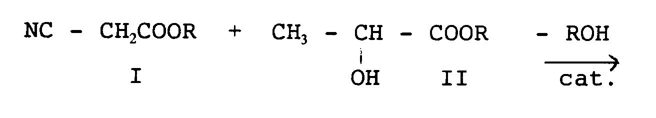 in which R denotes a C₁-C₁₀-hydrocarbon radical and 1 to 20 mol of cyanoacetic esters are used per mol of the lactic ester, in the presence of 0.001 to 0.1 mol of a basic catalyst per mol of lactic ester at 100 to 230°C, the corresponding alcohol being distilled off, and the reaction is terminated before achieving the theoretically possible amount of alcohol at a lactic ester conversion of 76 to 85% by destroying the catalyst and rendering it ineffective.

2. A process according to claim 1, characterized in that reaction temperatures of 140 to 200°C are chosen.

3. A process according to either of claims 1 and 2, characterized in that 1 to 10 and preferably 1.1 to 2 mol of cyanoacetic ester are used per mol of lactic ester.

4. A process according to any of claims 1 to 3, characterized in that alcoholates of alkali metals or of alkaline earth metals, preferably of Na, K, Mg, Sr or Ba, and, as alcohols, of C₁- to C₁₀- straight-chain or branched alkanols, preferably sodium methylate, ethylate or butylates or potassium methylate, ethylate or butylates, are used as catalysts.

5. A process according to any of claims 1 to 4, characterized in that 0.005 to 0.05, in particular 0.01 to 0.05, mol of catalyst is used per mol of lactic ester.

## Revendications

1. Procédé de fabrication d'esters d'acide 2-cyanoacétoxy-propionique de formule générale III, 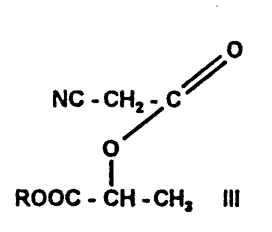 caractérisé en ce qu'
on fait réagir les esters de l'acide cyanoacétique de formule générale I en éliminant de l'alcool en présence d'esters d'acide lactique de formule générale II, 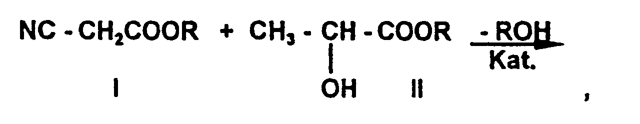 où R représente un reste hydrocarbure C₁-C₁₀ et on utilise 1 à 20 mol d'ester d'acide cyanoacétique par mode d'ester d'acide lactique, en présence de 0,001 à 0,1 mol d'un catalyseur basique par mol d'ester d'acide lactique entre 100 et 230°C en éliminant par distillation l'alcool correspondant, et on interrompt la réaction avant d'obtenir la quantité d'alcool théorique pour un taux de réaction de l'ester d'acide lactique de 76 à 85 % en détruisant le catalyseur et le rendant inefficace.

2. Procédé selon la revendication 1,
caractérisé en ce qu'
on choisit des températures de réaction de 140 à 200°C.

3. Procédé selon les revendications 1 et 2,
caractérisé en ce qu'
on utilise par mol d'ester d'acide lactique 1 à 10, de préférence 1,1 à 2 moles d'ester d'acide cyanoacétique.

4. Procédé selon les revendications 1 à 3,
caractérisé en ce qu'
on utilise comme catalyseurs des alcoolates de métaux alcalins ou de métaux alcalino-terreux, de préférence ceux de Na, K, Mg, Sr, Ba et comme alcools des alcanols C₁ à C₁₀ à chaîne droite ou ramifiée, de préférence du méthylate, de l'éthylate ou des butyrates de Na ou de K.

5. Procédé selon les revendications 1 à 4,
caractérisé en ce qu'
on utilise par mole d'ester d'acide lactique 0,005 à 0,05, notamment 0,01 à 0,05 mole de catalyseur.
